# EUROPEAN PATENT SPECIFICATION

(11) **EP 4 273 569 B1**
(45) Date of publication and mention of the grant of the patent: **02.07.2025**
(21) Application number: 22171617.8
(22) Date of filing: 04.05.2022
(51) Int. Cl.: G01R 33/56, G01R 33/565, G01R 33/50, G01R 33/3875

(54) **METHOD FOR GENERATING A SUBJECT-SPECIFIC MAP OF A TISSUE PROPERTY**
VERFAHREN ZUM ERZEUGEN EINER PATIENTEN-SPEZIFISCHEN KARTE EINER GEWEBEEIGENSCHAFT
PROCÉDÉ PERMETTANT DE GÉNÉRER UNE CARTE SPÉCIFIQUE À UN SUJET D'UNE PROPRIÉTÉ DE TISSU

(43) Date of publication of application: 08.11.2023
(73) Proprietor: Siemens Healthineers AG, 91301 Forchheim (DE)
(72) Inventor: Testud, Frederik, 22467 Lund (SE)
(74) Representative: Siemens Healthineers Patent Attorneys

(56) References cited:
- US-A1- 2012 076 378
- US-A1- 2014 296 696
- US-A1- 2015 362 578
- PROCEEDINGS OF THE INTERNATIONAL SOCIETY FOR MAGNETIC RESONANCE IN MEDICINE, ISMRM, JOINT ANNUAL MEETING ISMRM-ESMRMB, PARIS, FRANCE, 16-21 JUNE 2018 (PUB 01.06.2018), no. 1038, 1 June 2018 (2018-06-01), XP040700246
- MARQUES JOSÉ P ET AL: "Towards QSM Challenge 2.0: Creation and Evaluation of a Realistic Magnetic Susceptibility Phantom", PROCEEDINGS OF THE INTERNATIONAL SOCIETY FOR MAGNETIC RESONANCE IN MEDICINE, ISMRM, 27TH ANNUAL MEETING AND EXHIBITION, MONTRÉAL, QUÉBEC, CANADA, 11 MAY - 16 MAY 2019 (PUB 26.04.2019), 26 April 2019 (2019-04-26), pages 1122, XP055973811, Retrieved from the Internet <URL:https://cds.ismrm.org/protected/19MProceedings/PDFfiles/1122.html> [retrieved on 20221021]
- ZHOU J, AGHAEIFER A, BAUSE J, LOKTYUSHIN A, HAGBERG G, SCHEFFLER K: "Magnetic field estimation with ultrashort echo time (UTE) imaging", PROCEEDINGS OF THE INTERNATIONAL SOCIETY FOR MAGNETIC RESONANCE IN MEDICINE, ISMRM, 27TH ANNUAL MEETING AND EXHIBITION, MONTRÉAL, QUÉBEC, CANADA, 11 MAY - 16 MAY 2019 (PUB 26.04.2019), no. 4509, 26 April 2019 (2019-04-26), XP040711893
- ANDREW P. LEYNES ET AL: "Hybrid ZTE/Dixon MR-based attenuation correction for quantitative uptake estimation of pelvic lesions in PET/MRI", MEDICAL PHYSICS., vol. 44, no. 3, 16 March 2017 (2017-03-16), US, pages 902 - 913, XP055497672, ISSN: 0094-2405, DOI: 10.1002/mp.12122

## Description

The invention relates to a computer implemented method for generating a subject-specific map of a magnetic susceptibility of a region of interest within a subject, and a computer program and system.

In magnetic resonance imaging and spectroscopy, the homogeneity of the B₀ magnetic field is of crucial importance. However, as soon as an object, such as a part of a subject's body, is introduced into the main magnetic field, the field lines are distorted. This distortion, also referred to as demagnetization field, is determined by the magnetic susceptibility distribution within the object. Especially the interface between air and tissue, as is found for example in the human head because of the sinuses, leads to particularly high magnetic field inhomogeneities. Although every magnetic resonance (MR) scanner has an active shimming system using coils with adjustable currents, usually the demagnetization field can be compensated only partially by shimming.

If the magnetic susceptibility distribution Chi(r) in the body part to be examined is known, as well as its positional orientation within the static magnetic field B₀, then the effect of the demagnetization field can be corrected for, as shown for example in reference 9 (all references are listed at the end of the description).

Also, shimming generally does not allow for any dynamic changes of the orientation of the susceptibility distribution Chi(r) because of any subject motion. These effects may not be negligible and may lead to significant image degradation. In particular, for MR imaging sequences sensitive to off resonances, for example, gradient-echo echo planar imaging (GRE EPI) (see reference 7) or multi-TE GRE acquisitions as used for susceptibility weighted imaging (see reference 8), the effect of the demagnetization field needs to be taken into account.

The following prior art documents can be seen as background for the invention:
US 2012/076378 A1
US 2014/296696 A1
US 2015/362578 A1
Florkow et al: "The influence of different MRcontrasts in multi-channel convolutional neural networks on pseudo-CT generation for orthopedic purposes", PROCEEDINGS OF THE INTERNATIONAL SOCIETY FOR MAGNETIC RESONANCE IN MEDICINE, ISMRM, JOINT ANNUAL MEETING ISMRM-ESMRMB, PARIS, FRANCE, 16-21 JUNE 2018
Marques Jose P et al: "Towards QSM Challenge 2.0: Creation and Evaluation of a Realistic Magnetic Susceptibility Phantom", PROCEEDINGS OF THE INTERNATIONAL SOCIETY FOR MAGNETIC RESONANCE IN MEDICINE, ISMRM, 27TH ANNUAL MEETING AND EXHIBITION, MONTREAL, QUEBEC, CANADA, 11 - 16 MAY 2019, page 1122
Zhou J, Aghaeifer A, Bause J, Loktyushin A, Hagberg G, Scheffler K: "Magnetic field estimation with ultrashort echo time (UTE) imaging", PROCEEDINGS OF THE INTERNATIONAL SOCIETY FOR MAGNETIC RESONANCE IN MEOICINE, ISMRM, 27TH ANNUAL MEETING ANO EXHIBITION, MONTREAL, QUEBEC, CANAOA, 11 - 16 MAY 2019, no. 4509
Andrew P. Leynes et al: "Hybrid ZTE/Oixon MR-based attenuation correction for quantitative uptake estimation of pelvic lesions in PET/MRI", MEDICAL PHYSICS, vol. 44, no. 3, 16 March 2017, pages 902-913

However, at present no fast and sufficiently detailed methods for obtaining a subject-specific magnetic susceptibility distribution map are known.

It is therefore an object of the invention to provide a method for generating a subject-specific map of a magnetic susceptibility of a region of interest within the subject.

These objects are fulfilled by the method according to claim 1, a computer program according to claim 3 and a system according to claim 4.

According to one aspect, the invention is directed to a computer implemented method for generating a subject-specific map of the magnetic susceptibility of a region of interest within the subject, the method comprising the steps of:
- Receiving an MR input image of the region of interest;
- Feeding the MR input image into an algorithm, wherein the algorithm comprises at least one trained artificial neural network, wherein the output of the algorithm is an output image having improved contrast between bone and air, wherein the output image of the algorithm is a synthetic CT image;
- Segmenting the output image into air and at least two types of tissue, namely at least bone and at least one type of soft tissue, to obtain a segmented image;
- Assigning pre-determined values for the magnetic susceptibility to each category of tissue and air in the segmented image to obtain the subject-specific map of the magnetic susceptibility.

The invention is based on the insight that the different tissue types in the human body have different susceptibility values, which however can be estimated in advance, as they are not subject-specific, or only to a small degree. Therefore, if an image is segmented into the various tissue types and typical susceptibility values for each tissue type are assigned to the segmented regions, a susceptibility map can be obtained. However, it has so far not been possible to automatically segment MR images in this way, because small and thin structures (such as the bones constituting the sinuses) as well as air and bone do not generate a large or any MR signal and therefore appear exactly the same in MR images, namely having very low pixel values within the noise range, which are generally depicted as black. Therefore, if an MR image is segmented into different tissue types, it is difficult to differentiate between small structures, bone and air, especially in the head. On the other hand, also attenuation correction maps (AC-maps) consist of only a limited number of tissue types, namely lungs, soft tissue, fat and bones. AC-maps are necessary for a correct reconstruction of PET volumes. Several methods have been proposed to estimate AC-maps from MR images, e.g., as described in reference 16, where an ultrashort echo time (UTE) sequence is used to derive the AC-map. Such MR images can be acquired simultaneously to the PET experiments with a PET-MR, instead of AC-maps derived from a separately acquired CT volume. However, these techniques, which provide a distribution of air cavities, bone and tissue, can still wrongly assign spatial positions to tissue instead of bone, or to tissue instead of air cavities, especially in the head.

The invention therefore uses an MR input image of the region of interest and applies an algorithm thereto, wherein an output image having improved contrast between bone and air is generated. According to the invention, the algorithm comprises at least one trained artificial neural network (NN). The neural network may be a convolutional neural network, for example having 4 to 16 convolutional layers. The convolutional neural network may be based on an encoder-decoder structure with symmetric concatenations between corresponding states, for example of the type as described in reference 19. For example, each layer in the neural network may comprise one or more 3x3x3 kernels, batch normalisation, an activation function, for example a rectified linear unit, and a dropout layer in the encoding part, and vice versa in the decoding part. The NN may have been trained by a machine learning algorithm, for example by a back-propagation method. The NN may have been trained using real images, for example if the output image is a pseudo CT image, the NN may have been trained using MR images of a body part as input training data, and real CT images of the same body part as output training data.

The MR input image is preferably a three-dimensional (3D) image, although a stack of two-dimensional (2D) images may also be used to estimate the magnetic susceptibility map. The MR input image may also comprise several images of the region of interest, in particular several images which have been acquired in one imaging sequence, for example in-phase and opposed-phase images obtained by chemical shift imaging. The method of the invention may also be applied to a time series of MR input images, for example a series of EPI images which are acquired during a fMRI (functional magnetic resonance imaging) experiment, in which a series of images, such as EPI images, is acquired in order to observe changes in the subject's brain activity over time, in particular the change in BOLD-contrast (Blood Oxygen Level Dependent). In this case, the method of the invention may be used to obtain a time series of susceptibility maps and thus enable correction of the shimming or imaging parameters during the fMRI experiment, to ensure the best image quality.

In an embodiment, the neural network is trained on the specific body part examined, for example the human head, prostrate, kidney, abdomen, lung, heart of specific organs or limbs. The algorithm comprising the at least one neural network may therefore be chosen according to the body part in which the region of interest is situated. By this smart use of artificial intelligence, the invention can make use of (preferably convolutional) neural networks to improve the contrast between bone and air, which otherwise does not allow suitable segmentation of the images.

The output image is then segmented into air and at least two types of tissue, namely at least bone and at least one type of soft tissue (or soft tissue in general), to obtain a segmented image. In the simplest form, the segmentation differentiates between bone, air and soft tissue, wherein soft tissue comprises all types of soft tissue, e.g. fat, muscle, white matter, grey matter, skin, liver, etc.. In more sophisticated embodiments, the soft tissue may also be differentiated into several different types, for example fat and other tissue. The segmentation step may use thresholding, 3D adaptive thresholding, model-based segmentation, atlas-based segmentation, histogram-based methods, edge detection, region-growing, or any other segmentation methods. The central assumption of model-based approaches is that the structures of interest have a tendency towards a particular shape. Therefore, one can seek a probabilistic model that characterizes the shape and its variation. When segmenting an image, constraints can be imposed using this model as a prior, as described in reference 38.

In the next step, predetermined values for the magnetic susceptibility are assigned to each pixel or voxel in the segmented image, according to its type of material (bone, soft tissue or air), to obtain the subject-specific map. The values used for the magnetic susceptibility can be for example -9.2 ppm for soft tissue, - 8.44 ppm for bone and 0.36 ppm for air, as disclosed in reference 11 or 12. However, other predetermined values may also be used.

It has been shown that even this simplified map yields very good results in calculating the corresponding demagnetization field, since it is entirely subject-specific, without making use of any atlases or other preconceived ideas about the patient's anatomy. The only predetermined values are the susceptibility values of the different tissue types, which however are known and do not vary significantly from patient to patient.

The method of the invention is preferably performed by a computer, which may be any processing unit such as a CPU and/or a GPU of a server or PC, or preferably a processing unit within an MR scanner. It may be performed on any computing device including a cloud computer, a laptop, tablet computer or other mobile device. The method is preferably carried out during an MR examination of a subject, i.e., while the subject is in the sensitive region of the MR scanner. This is because the generated tissue property map may be used to improve the quality of further MR acquisitions. The MR scanner may also be a PET/MR scanner or other hybrid device. The subject may be a human or animal, in particular a patient during an MRI examination.

According to the invention, the output image of the neural network is a synthetic CT (Computed Tomography) image. Such a method for transforming a low-resolution MR input image into a high-resolution MR image using a CNN based network, and transforming the high-resolution MR image to a pseudo-CT image has been proposed in US 2020/003 4948 A1 (reference 20). In this method, two convolutional neural networks are used one after the other, and the output image is again suitable for segmenting into the different tissue types, because CT images distinguish very well between bone and air, so a segmentation into soft tissue, bone and air does not provide any problems. Also, CT images have a high resolution, and a CNN may be trained to achieve such high resolution, even from lower resolution MR input images.

The MR input image of the region of interest may be acquired by any possible MR sequence, for example spin echo, fast spin echo, gradient echo, echo planar imaging (EPI) etc. The MR input image may be three-dimensional (3D) or two-dimensional (2D), wherein the method may also be applied to a stack of 2D images. The MR input image may also comprise several images, such as in-phase and opposed-phase, or a time series of images. According to an embodiment, the MR input image has been acquired using a fast or a standard MR imaging protocol, preferably Dixon, MPRAGE, MP2RAGE, MP3RAGE or sequences with ultrashort echo time (UTE). The MR input image can be an image that is acquired also for diagnostic purposes during a patient examination. It is advantageous if the MR input image is acquired within less than 3 minutes, preferably 30 seconds to 2 minutes, and the above-mentioned imaging methods allow an acquisition of a 3D image volume in about 10-60 seconds, preferably 20-40 seconds. A high-resolution 3D Dixon VIBE protocol may be used with CAIPIRINHA acceleration. As an example, such a protocol may take 39 seconds. The acquisition may be made even shorter if an acquisition with Compressed Sensing is used, and/or the resolution is reduced. A further advantage of UTE, MPRAGE and Dixon sequences is that they are available for MR scanners at all field strengths ranging from 1.5 T to 7 T, for example for 1.5 T, 3 T and 7 T MR scanners. A further advantage is that MR input images acquired using UTE and Dixon at 3 T have a good suitability to be converted for example into AC-maps using convolutional neural networks, as demonstrated in references 19 and 29.

According to an embodiment, the region of interest is at least partially within the head of the subject. As mentioned above, the human head poses particular challenges to MR protocols which are sensitive to be B₀ field inhomogeneities, because of the many air cavities in the head. Therefore, the use of an accurate magnetic susceptibility map can yield significant improvements in image quality. In other embodiments, the region of interest may at least partially include the human prostate, the kidney, the heart, or indeed any other organ or body part.

According to an embodiment, the method comprises the further step of estimating a map of the demagnetization field caused by the subject when placed in a B₀ magnetic field, wherein the estimation is done based on the magnetic susceptibility map. The demagnetization field B_{d} is the magnetic field generated by an object with a given magnetisation and is related to the susceptibility distribution of the object and the static magnetic field strength. It can be estimated by the convolution of the magnetic susceptibility distribution/map with the magnetic dipole and the Lorentz sphere correction, as described in references 3 and 4. Similar methods may be used to calculate a map of the demagnetization field from the susceptibility map and the B₀ magnetic field, as described for example in reference 12. This corresponds to a calculation of the magnetic field inhomogeneity induced by the presence of the part of the subject in the B₀ field. The demagnetization field map depends also on the exact orientation of the susceptibility map within the field of view.

The proposed invention can make use of convolutional neural networks (CNN's) and the possibility to segment the tissue types of interest, as described in references 9 and 11, and assigning them their corresponding susceptibility values. This approach is then much simpler and quicker than to perform the method proposed in reference 11. It only requires moderate computational power in a reasonable amount of time, wherein a modern GPU is already sufficient. The proposed approach may avoid using an atlas, it only relies on MR measurements from one or several acquisitions.

In the following, novel uses of a thus obtained magnetic susceptibility map are described. The different uses described herein may be applied cumulatively in one MR imaging session.

Generally, according to a further aspect, the use of a magnetic susceptibility map generated by the method described above to improve the quality of further MR acquisition from the region of interest can be contemplated. Preferably, these further MR acquisitions take place in the same MR examination session, although the magnetic field map may also be used in later MR examination sessions, once it has been registered with the position of the respective body part during that later session.

The magnetic suitability map can also be used to improve the homogeneity of a B₀ magnetic field of an MR scanner in which the subject is placed for MR acquisition, by estimating a map of the demagnetization field caused by the subject when placed in the B₀ magnetic field from the magnetic susceptibility map, and adjusting the shim coefficients of the MR scanner to compensate for the demagnetization field. Thereby, the standard shimming using the shim coils is improved, because the demagnetising field map contains information also in regions where no MR signal is available and has potentially a higher resolution than the field maps which are usually acquired in the context of shimming. Thus, more voxels can be used for fitting the shim coefficients.

Furthermore, the quality of MR acquisition from the region of interest may be improved by tracking any motion of the region of interest during the acquisition of an MR input image, applying the tracked motion to the magnetic susceptibility map to obtain a dynamic magnetic susceptibility map, estimating a dynamic demagnetization field map from the dynamic magnetic susceptibility map, and adjusting the shim coefficients in real time to compensate for the dynamic demagnetization field.

Subject motion is a major problem for magnetic resonance imaging. Prospective motion correction methods use an external device to track the subject's position, for example a tracking target comprising reflective markers and which are being watched by tracking cameras, as described in reference 1. Such a tracking system is capable of reporting positions and orientations of several tracking targets attached to the patient and thereby record or track patient motion in real time. This information may be used to update the position of the imaging volume prior to every excitation of the spin system. Other methods for tracking the motion of the region of interest include image-based tracking, such as described in reference 2. According to this method, a rigid body estimation of head movements is obtained by image-based motion detection. Both of these methods allow to follow the position and orientation of the region of interest during the acquisition of an MR image. This motion, which may preferably be a rigid motion including three translational and three rotational degrees of freedom, but which may also have more degrees of freedom, may be applied to the magnetic susceptibility map during the MR acquisition to obtain a dynamic magnetic susceptibility map. In this context, "dynamic" means that the map is available for different time points, i.e. it is a time sequence of magnetic susceptibility maps which is generated for the time span of the MR input image acquisition, during which the motion is tracked. For example, the tracked translational and rotational movements are imposed on the susceptibility map at each time point during the MR acquisition, with a pre-determined temporal resolution. Preferably in real time, e.g. at the pre-determined temporal resolution, a dynamic demagnetization field map is estimated from the dynamic magnetic susceptibility map, by the methods described herein. This map of the demagnetization field therefore takes into account any patient motion and is preferably generated in real time during the MR input image acquisition. According to this aspect, it may be used to adjust the shim coefficients in real time to compensate for the respective dynamic demagnetization field. Thereby, an excellent image quality is obtained even for moving subjects.

Furthermore, the magnetic susceptibility map may be used for motion correction of an MR image acquired with an imaging sequence from the region of interest by tracking any motion of the region of interest during the acquisition of an MR image; applying the tracked motion to the magnetic susceptibility map to obtain a dynamic magnetic susceptibility map; estimating a dynamic demagnetization field map from the dynamic magnetic susceptibility map; and adjusting the imaging sequence in real time to compensate for the dynamic demagnetization field. Again, the motion tracking may be performed either using image-based tracking or using an external tracking device, for example an optical tracking device. The further steps including generating a dynamic magnetic susceptibility map and therefore a dynamic demagnetization field may be performed as described above. In the final step, the imaging sequence is adjusted in real time to compensate for the dynamic magnetisation field. In other words, the dynamic demagnetization field map can be used for dynamic geometric distortion correction, for example for EPI and other sequences sensitive to the inhomogeneous field and its changes, using rotations and translations for body regions which are moving during the MR exam, and which information may be made available by the herein described methods. The encountered motion during the acquisition can be applied to the susceptibility distribution. More complex nonrigid deformations for the body region of interest may also be tracked. Also in this case, the shim magnetic field may be taken into account.

Furthermore, a dynamic demagnetization field map may be obtained as described herein above, and may be used for retrospective motion correction of an acquired MR image, which may be the MR input image, but may also be an MR image acquired afterwards during the same patient examination from the same or overlapping field of view. A suitable method is for example described in reference 22, WO 2011/127942 A1. This patent also uses a computed field inhomogeneity, which however is calculated from a susceptibility model which is less detailed than the susceptibility map proposed herein. The dynamic magnetisation field may be in particular used for correcting image distortions and/or intensity modulations. Thereby, the image quality of MR images of moving subjects may be improved. Using this method, even EPI imaging may be used, which is very sensitive to B₀ distortions. If a time series of MR images is acquired, the dynamic demagnetization field map may be used to correct the geometric image distortions at each time point during the acquisition, in particular for each MR image in the time series.

Furthermore, the magnetic susceptibility map may be used to improve the quality of dynamic chemical exchange saturation transfer (CEST) image acquisition of the region of interest by tracking any motion of the region of interest, applying the tracked motion to the magnetic susceptibility map, estimating a dynamic demagnetization field map and correcting the dynamic CEST images by using the dynamic demagnetization field map. Since CEST imaging is based on the continuous transfer of excited ¹H protons from one chemical species to the surrounding water, leading to a build-up of saturation in water, it requires frequency selective excitation of protons and therefore a high homogeneity of the B₀ magnetic field. A knowledge of the demagnetization field map can be used to shift back the z-spectrum. In other words, knowledge of the off-resonance frequency shift per voxel is required so that all the measurements can be shifted back to their designated spectral positions.

Furthermore, the magnetic susceptibility map can also be used to improve the quality of dynamic CEST image acquisition of the region of interest by tracking any motion of the region of interest during the acquisition; applying the tracked motion to the magnetic susceptibility map to obtain a dynamic magnetic susceptibility map; estimating a dynamic demagnetization field from the dynamic magnetic susceptibility map; and correcting the dynamic Chemical Exchange Saturation Transfer images by using the dynamic demagnetization field map.

In other words, the inventive susceptibility map also allows to improve the quality of dynamic CEST images. Z-spectra estimated voxel-wise from CEST acquisitions, typically gradient echo acquisitions, need to be corrected for B₀ inhomogeneity at the corresponding voxel. When dynamic CEST experiments are performed, as for example in glucose CEST described in references 24 and 25, then it is necessary to take the time variations of the magnetic inhomogeneity field into account, which can be dependent of subject motion. Again, an accurate subject-specific susceptibility model which can be varied with the subject's position will improve the correction for the field inhomogeneity.

Furthermore, the use of a magnetic susceptibility map in the calculation of radio-frequency (RF) excitation pulses of a multi-transmit system of an MR scanner for a given excitation trajectory for parallel excitation can be contemplated. In parallel excitation, a transmit system having several RF antennas (i.e. a multi-transmit system), for example an array or phased array of RF coils, is used to produce a complex RF pulse. For the same RF pulses, gradient fields are applied during the RF excitation, allowing specific desired trajectories of the magnetisation in transmit k-space. To realize these trajectories, an accurate knowledge of the magnetic field is important, see reference 31. Therefore, a subject-specific demagnetization field can be used to improve the calculation of RF excitation pulses.

Furthermore, the subject-specific demagnetization field map can be used instead of a standard field map in advanced image reconstruction methods. Thus, after the method for generating a subject-specific map of a tissue property has been carried out, a map of the demagnetization field is calculated from the magnetic susceptibility map. Optionally, a further MR image is then acquired while the subject is placed with the MR Scanner, and the demagnetization field is used in the reconstruction of the acquired image. Preferably, image reconstruction involves advanced image reconstruction methods, such as iterative image reconstruction using an MR forward model, as described for example in reference 33 or 35. The image reconstruction method of ref. 35 includes a retrospective motion correction by including motion operations into the MR forward model, wherein the MR forward model is essentially a mathematical description (e.g. an encoding matrix) of the operation by which the MR image is generated from the proton distribution. In case of multi-channel k-space data acquired in a multi-shot imaging protocol, the MR forward model may be described by an encoding matrix, which includes the effects of rigid-body motion for each shot, Fourier encoding, and optionally subsampling and coil sensitivities of the multi-channel coil array. The solving of such an MR forward model usually requires several optimization iterations. The SENSE (SENSitivity Encoding) forward model was introduced by reference 36. An MR forward model used in advanced, in particular iterative, image reconstruction may include the effect of the demagnetization field calculated from a susceptibility map, which has been generated according to the inventive method. This reduces the geometric distortions of the reconstructed image.

Furthermore, the demagnetization field may be used in the reconstruction of MR data acquired using compressed sensing (e.g. described in reference 37) to improve the reconstructed images with regard to geometric distortion, as described in reference 5. Compressed sensing typically involves randomized subsampling of k-space.

Furthermore, the subject-specific demagnetization field map can be used in quantitative susceptibility mapping, in particular in one or several steps of the post-processing pipeline for quantitative susceptibility mapping (QSM), which are summarized in references [26] and [27]. Typically (but not limited to), a 3D multi-TE GRE acquisition is used for the acquisition and no signal is available in regions consisting of bones. The QSM pipeline can be separated in several steps. One of the steps comprises removing the background field of the frequency map obtained from the acquired data over a masked region of the brain or the body part in question (the masked region is defined where MR signal is sufficient, e.g. above noise level) to obtain a background field corrected frequency map. The subject-specific demagnetization field map in this step can lead to an improved background field corrected frequency map, because magnetic field information outside of the brain or the body part is available. In a final step of the QSM post-processing pipeline, the susceptibility distribution is estimated from the masked background field corrected frequency map which can be achieved numerically by solving the ill-posed problem of deconvoluting the masked background field corrected frequency map with the unit magnetic dipole response. The additional knowledge of having a subject-specific susceptibility distribution helps to improve the condition of the ill-posed problem by, for example, generating a simulated demagnetization field in at least two additional different orientations of the susceptibility distribution to the acquired 3D multi-TE GRE image, similar as in reference [34]. Another possibility is to further constrain the inverse ill-posed problem by including the susceptibility distribution in the numerical algorithm.

The invention is also directed to a computer program comprising instructions which, when the program is executed by a computer, cause the computer to carry out the method for generating a subject-specific map of a magnetic susceptibility. The computer may be any processing unit, cloud computer or server, also a processing unit on a mobile device such as a laptop, smartphone or tablet.

The computer program can be stored on a non-transient digital storage medium. Such digital storage medium may be a hard disk, optical disk, magnetic storage medium, SD card, USB stick, CD-ROM or any possible portable or nonportable data storage medium.

The invention is also directed to a system for generating a subject-specific map of a magnetic susceptibility of a region of interest within a subject, the system comprising: a first interface, configured for receiving an MR image of the region of interest; a computational unit, configured for
- feeding the MR image as input into an algorithm, wherein the algorithm comprises at least one trained artificial neural network, wherein the output of the algorithm is an output image having improved contrast between bone and air, wherein the output image of the algorithm is a synthetic CT image;
- segmenting the output image into air and at least two types of tissue, namely at least bone and at least one type of soft tissue, to obtain a segmented image;
- assigning pre-determined values for the magnetic susceptibility to each category of tissue and air in the segmented image to obtain the subject-specific map of the magnetic susceptibility; and
a second interface, configured for providing the map of the magnetic susceptibility.

The system may be part of an MR scanner, and particular part of the computer controlling the MR scanner and the image acquisition. However, the system may also be a remote computer to which the MR image is transferred.

The invention will now be illustrated by means of embodiments with reference to the attached drawings. In the drawings
- Fig. 1: shows a flowchart of an embodiment of a method which is outside the scope of the present invention.
- Fig. 2: shows a flowchart of an embodiment of the method of the invention.
- Fig. 3: shows a flowchart of a trained neural network according to an embodiment of the invention.
- Fig. 4: shows a schematic illustration of a trained neural network.
- Fig. 5: shows a flowchart of an embodiment of a method which is outside the scope of the present invention.
- Fig. 6: shows a flowchart of a first possible use of a subject-specific susceptibility map.
- Fig. 7: shows another possible use of a subject-specific susceptibility map.
- Fig. 8: shows a system according to an embodiment of the invention.
- Fig. 9: shows another possible use of a subject-specific susceptibility map.

Similar features are designated with the same reference numbers in the following.

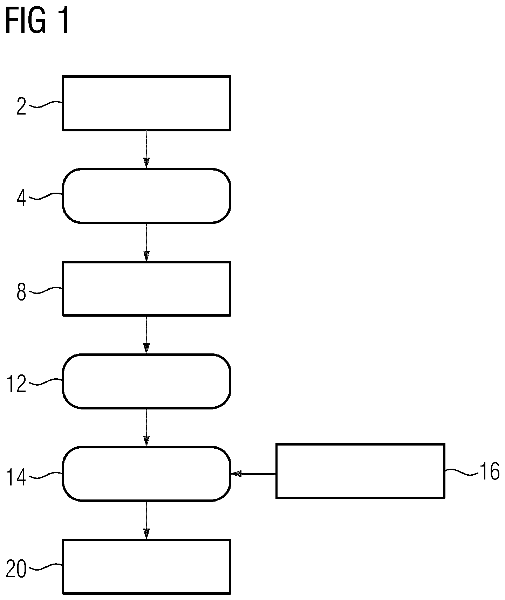

Figure 1 illustrates a method outside the scope of the present invention for generating a subject-specific map of a tissue property, in this instant the magnetic susceptibility of a region of interest. As a starting point, an MR image 2 of the region of interest is fed into a trained neural network 4, wherein the MR image 2 may for example be acquired using a localiser-like sequence using a fast imaging protocol. The neural network 4 converts this MR image 2 into a synthetic AC-map 8. The AC-map 8 may have the same or higher resolution than the input MR image and generally has better contrast between bone and air. In a next step 12, the AC-map 8 is segmented into at least two types of tissue, namely at least bone and a type of soft tissue, and air. For example, this step may result into a segmented image in which every voxel is assigned to one of the above tissue types or air. In the next step 14, predetermined values for the tissue property, in particular the magnetic susceptibility, is assigned to each type of tissue and air in the segmented image. These predetermined values are for example known from reference 11 and may be stored in a computer or database 16. This step already results in the subject-specific susceptibility map 20. For the purposes of estimating the magnetic field inhomogeneity caused by the subject with this susceptibility map being placed in the B₀ field, the segmented susceptibility image 20 is more than sufficient. It is more accurate to calculate the field distortions from a magnetic susceptibility map, than to measure them directly, and has the advantage over other MR-based methods that values are available in areas from which no MR signal can be obtained, such as from regions of air inside and outside the body. The susceptibility map 20 will be the more accurate, the more tissue types are being segmented.

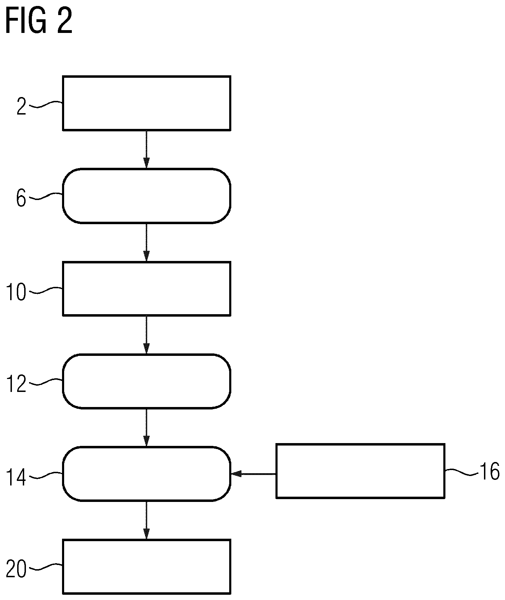

Figure 2 shows a method according to the invention, in which the MR image 2 is fed into a different type of trained neural network 6, which has as output image a synthetic CT image 10. Again, the synthetic CT image 10 may have high resolution and particular good contrast between bone and air, better than the input MR image. In the next step 12, the synthetic CT image 10 is segmented into different tissue types and air. In the next step 14, again predetermined values for the magnetic susceptibility are assigned to each voxel and thereby the magnetic susceptibility map 20 is generated.

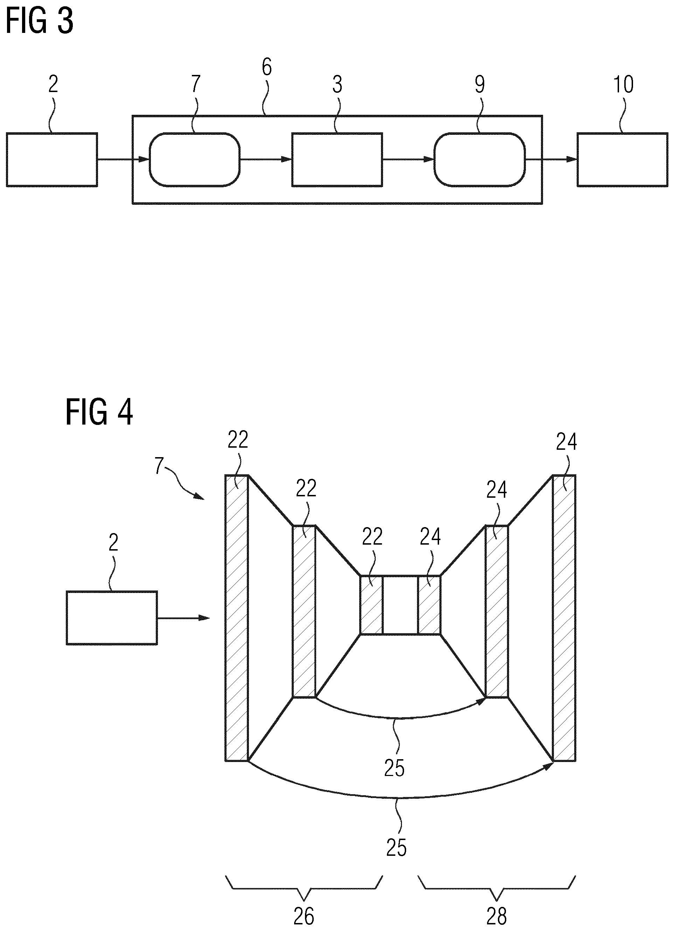

Figure 3 illustrates the trained neural network according to an embodiment in more detail. In this embodiment, the neural network 6 actually comprises two neural networks, wherein the first neural network 7 gives as output an MR image 3 of higher resolution than the MR input image 2. In the next step 9 performed by another neural network, a synthetic CT image 10 is generated.

Figure 4 illustrates a trained convolutional neural network, which may be used for the conversions described herein, it may for example be the neural network 7, but may also be an example for a neural network 4 or 8. In detail, the neural network takes the MR input image 2 (which may include several MR images) as input layer and comprises a number of layers 22, 24 between the input layer and the output layer to allow deep learning. According to a preferred embodiment, the convolutional neural network 7 comprises an encoder portion 26 and a decoder portion 28. The encoder portion 36 comprises a number of layers 22, for example two to six such layers. Each layer 22 comprises the operation of one or several convolutional kernels, for example 3x3x3 kernels for a 3D input image 2, followed by an activation function, followed by a down-sampling/pooling operation, for example a MAX function. Thereby, the size of the layers is reduced going from one layer to the next in the encoder portion. Usually, several convolutions are applied, so that the layers have more and more channels from one layer 22 to the next. There may be one or several layers, which may also be fully connected layers, in the central portion between the encoder part 26 and the decoder part 28. The decoder part 28 comprises also e.g., two to eight layers 24, generally the same number of layers as the encoder portion. Each layer 24 also comprises a convolution kernel, an activation function and an up-sampling operation, so that the output layer may have the same dimensions as the input layer, although it may also have a higher or lower dimension. In addition, the neural network may comprise skip connections 25 by which the more detailed information from the encoder layers 22 is directly fed into the layers 24 of the decoder portion 28. Thereby, the central layers of the CNN are skipped.

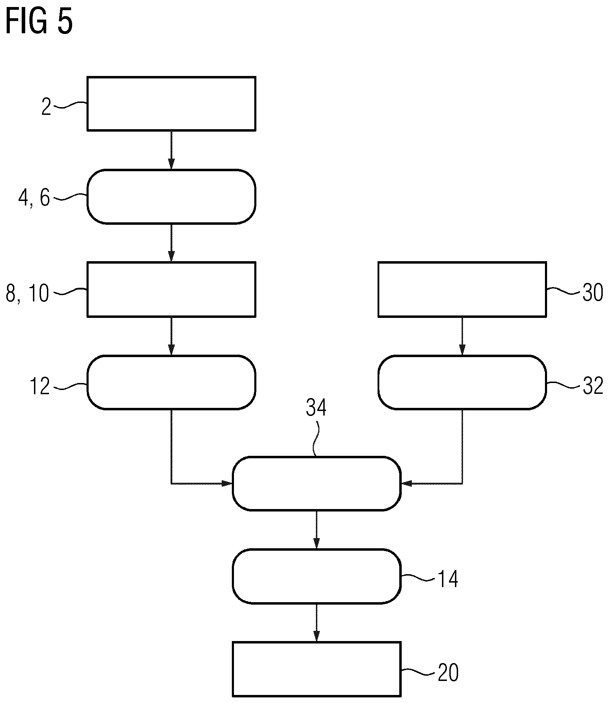

Figure 5 illustrates a method outside the scope of the present invention, in which a morphological image 30 is used. The left side is the same as the embodiments of Figures 1 and 2 and includes neural networks 4 or 6 generating synthetic AC-maps or CT images 8, 10, followed by segmenting these images in step 12 into at least bone, soft tissue and air. On the right side, in addition a morphological MR image 30 of the region of interest is obtained, either from a previous or the same session, and it is used to segment different types of soft tissue in step 32, for example white matter, grey matter, skin and CFS for a head image. In step 34, the soft tissue segmentation 32 is combined with the segmentation of bone, soft tissue and air in segmented image 12, so that the improved segmented image comprises more soft tissue types. This improved segmented image is then assigned predetermined susceptibility values for each voxel in step 14 to yield the susceptibility map 20.

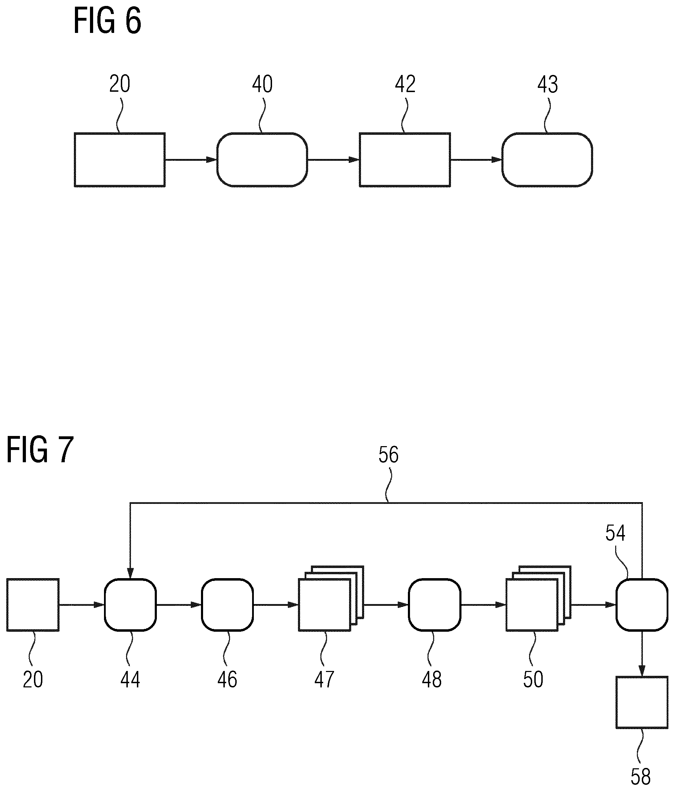

Figures 6 and 7 illustrate possible uses of a susceptibility map generated according to embodiments of the invention. According to figure 6, for example, the susceptibility map 20 is used to estimate a map of the demagnetization field caused by the subject when placed in a B₀ magnetic field in step 40. This demagnetization field map 42 is more accurate than any field map estimated by previous methods and can be used for example to adjust the shim coefficients of the MR scanner in step 43. Alternatively, step 43 may include correcting the z-spectra obtained from CEST imaging for each voxel. Further alternatively, in step 43, RF excitation pulses may be calculated with more accuracy. Last but not least, the demagnetization field map 42 can be used for iterative image reconstruction.

The susceptibility map 20 may also be used for dynamic corrections, as illustrated in figure 7. In this embodiment, in step 44, the motion of the subject is observed or tracked by known methods, for example by tracking a motion marker or tracking target which is attached to the subject close to the region of interest. This may be carried out during the execution of an MR sequence, e.g. an imaging sequence, for acquiring MR data. In the next step 46, this tracked motion, which comprises for example changes in the translation and orientation of the object from one time point to the next, wherein the time resolution may correspond to the RF excitation pattern of the MR sequence, is applied to the susceptibility map 20 to obtain a dynamic susceptibility map 47. In step 48, a dynamic demagnetization field map 50 is estimated from the dynamic magnetic susceptibility map 47. This dynamic demagnetization field 50 map may then be used for example to adjust the MR sequence, or to adjust the shim currents in step 54. This is repeated in real time, as indicated by arrow 56, until the MR image acquisition is completed and a motion corrected and/or distortion corrected MR image 58 is generated.

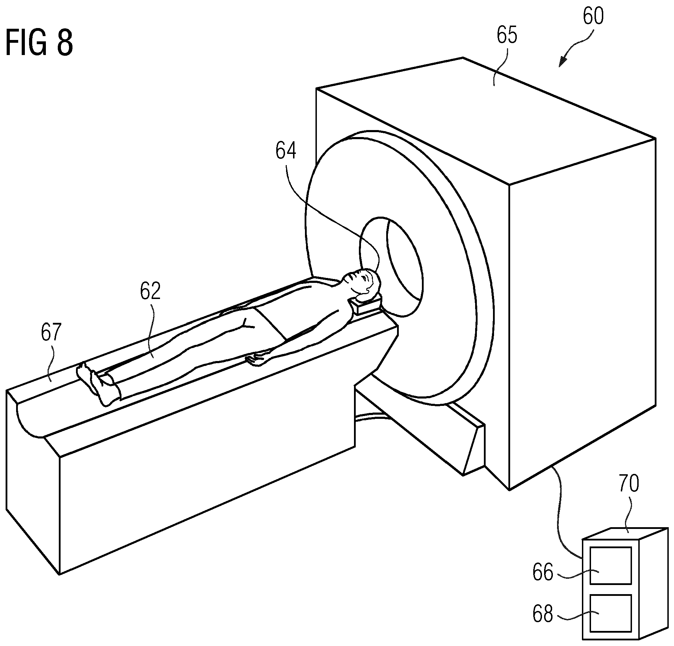

Figure 8 illustrates a system 60 for carrying out the method according to the invention. The system 60 in this case is an MR scanner comprising a main magnet 65 and a patient bed 67. A patient 62 is lying on the patient bed, while its head 64 is being imaged. The MR scanner 60 is connected to a computer 70 comprising at least a CPU 66 and a data storage 68. The computer 70 may be the computer generally controlling the MR acquisition of the MR scanner 60 and is adapted to perform the method.

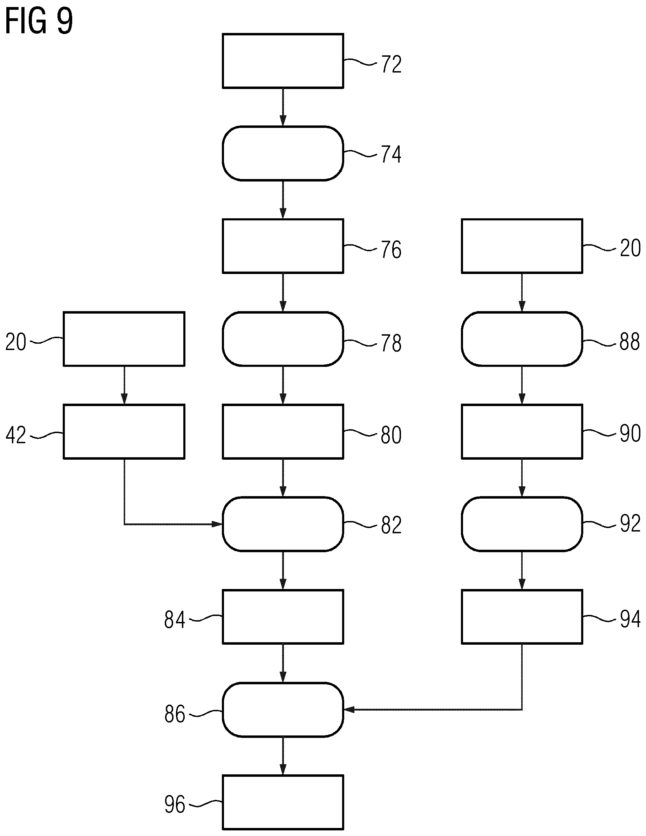

Figure 9 shows a flow diagram illustrating the use of the subject specific susceptibility map in quantitative susceptibility mapping (QSM). In step 72, multi-channel and optionally multi-echo MR data, as acquired using an imaging protocol for QSM, for example as disclosed in references 26 and 27, are received. In a next step 74, data from the different receiving coil channels are combined and phase unwrapping is performed, resulting in combined data 76. In the next step 78, a frequency map 80 is extracted from the combined data. In step 82, the background field is removed from the frequency map 80 using a subject specific demagnetization field map 42 derived from a segmented susceptibility image 20, which has been derived according to the method described herein. Step 82 results in a background field corrected frequency map 84. To carry out the next step 86, which is a field-to-source inversion, a demagnetisation field map 42 is required in several orientations. This data is also derived from the segmented susceptibility image 20, which is rotated by at least 2 additional rotations in step 88, resulting in the rotated segmented susceptibility images 90. From all of these susceptibility maps 90, demagnetisation field maps 94 in several orientations are estimated in step 92. This corresponds to step 40 in figure 6. Accordingly, the demagnetisation field maps in several orientations 94 are combined with the background field corrected frequency maps 84 in step 86, the field-to-source inversion. Step 86 results in the QSM map 96.

### Cited References

[1] M. Zaitsev, C. Dold, G. Sakas, J. Hennig, and O. Speck, "Magnetic resonance imaging of freely moving objects: prospective real-time motion correction using an external optical motion tracking system.," Neuroimage, vol. 31, no. 3, pp. 1038-1050, Jul. 2006.
[2] S. Thesen, O. Heid, E. Mueller, and L. R. Schad, "Prospective Acquisition Correction for head motion with image-based tracking for real-time fMRI," Magn. Reson. Med., vol. 44, no. 3, pp. 457-465, 2000.
[3] K. M. Koch, X. Papademetris, D. L. Rothman, and R. A. de Graaf, "Rapid calculations of susceptibility-induced magneto-static field perturbations for in vivo magnetic resonance.," Phys Med Biol, vol. 51, no. 24, pp. 6381-6402, Dec. 2006.
[4] R. Salomir, B. Denis de Senneville, and C. T. Moonen, "A fast calculation method for magnetic field inhomogeneity due to an arbitrary distribution of bulk susceptibility.," Concepts Magn Reson B Magn Reson Eng, vol. 19B, no. 1, pp. 26-34, 2003.
[5] P. Jezzard and R. S. Balaban, "Correction for geometric distortion in echo planar images from B0 field variations.," Magn Reson Med, vol. 34, no. 1, pp. 65-73, Jul. 1995.
[6] J. Maclaren, M. Herbst, O. Speck, and M. Zaitsev, "Prospective motion correction in brain imaging: A review.," Magn Reson Med, vol. 69, no. 3, pp. 621-636, 2013.
[7] P. Mansfield, "Multi-planar image formation using NMR spin echoes.," J Phys C Solid State Phys, vol. 10, no. 3, pp. L55-L58, 1977.
[8] J. Duyn, "MR susceptibility imaging," J. Magn. Reson., vol. 229, pp. 198-207, 2013.
[9] R. Boegle, J. Maclaren, and M. Zaitsev, "Towards combining prospective motion correction and distortion correction for EPI," Proc. 18th Sci. Meet. Int. Soc. Magn. Reson. Med., vol. 34, no. 1995, p. 496, 2010.
[10] R. Boegle, J. Maclaren, and M. Zaitsev, "Combining prospective motion correction and distortion correction for EPI: towards a comprehensive correction of motion and susceptibility-induced artifacts.," Magn Reson Mater Phys Biol Med, vol. 23, no. 4, pp. 263-273, 2010.
[11] R. Sostheim, J. Maclaren, F. Testud, and M. Zaitsev, "Predicting Field Distortions in the Human Brain Using a Susceptibility Model of the Head," 2012, p. 3386. [
[12] J. F. Schenck, "The role of magnetic susceptibility in magnetic resonance imaging: MRI magnetic compatibility of the first and second kinds.," Med Phys, vol. 23, no. 6, pp. 815-850, Jun. 1996.
[13] C. M. Collins, B. Yang, Q. X. Yang, and M. B. Smith, "Numerical calculations of the static magnetic field in three-dimensional multi-tissue models of the human head.," Magn Reson Imaging, vol. 20, no. 5, pp. 413-424, Jun. 2002.
[14] P. E. Kinahan, B. H. Hasegawa, and T. Beyer, "X-ray-based attenuation correction for positron emission tomography/computed tomography scanners," Semin. Nucl. Med., vol. 33, no. 3, pp. 166-179, 2003.
[15] P. E. Kinahan, D. W. Townsend, T. Beyer, and D. Sashin, "Attenuation correction for a combined 3D PET/CT scanner," Med. Phys., vol. 25, no. 10, pp. 2046-2063, 1998.
[16] V. Keereman, S. Vandenberghe, Y. De Deene, R. Luypaert, T. Broux, and I. Lemahieu, "MRbased attenuation correction for PET using an ultrashort echo time (UTE) sequence," IEEE Nucl. Sci. Symp. Conf. Rec., pp. 4656-4661, 2008.
[17] T. Koesters et al., "Dixon sequence with superimposed model-based bone compartment provides highly accurate PET/MR attenuation correction of the brain," J. Nucl. Med., vol. 57, no. 6, pp. 918-924, Jun. 2016.
[18] C. N. Ladefoged et al., "Region specific optimization of continuous linear attenuation coefficients based on UTE (RESOLUTE): Application to PET/MR brain imaging," Phys. Med. Biol., vol. 60, no. 20, pp. 8047-8065, 2015.
[19] C. N. Ladefoged et al., "AI-driven attenuation correction for brain PET/MRI: Clinical evaluation of a dementia cohort and importance of the training group size," Neuroimage, vol. 222, p. 117221, Nov. 2020.
[20] P. Chunjoo, S. Mutic, H. Zhang, and O. Green, "ML-BASED METHODS FOR PSEUDO-CT AND HR MR IMAGE ESTIMATION," US 2020/0034948 Al.
[21] Y. Haibo and L. Shu, "PET image attenuation correction method and device, computer equipment and storage medium," CN111105472A, 2020.
[22] J. Maclaren, R. Bögle, and M. Zaitsev, "METHOD FOR CORRECTING SUSCEPTIBILITY-INDUCED IMAGE ARTIFACTS IN MRI AFTER PROSPECTIVE MOTION CORRECTION," WO 2011/127942 A1, 2011.
[23] K. M. Ward, A. H. Aletras, and R. S. Balaban, "A New Class of Contrast Agents for MRI Based on Proton Chemical Exchange Dependent Saturation Transfer (CEST)," J. Magn. Reson., vol. 143, no. 1, pp. 79-87, 2000.
[24] X. Xu et al., "Dynamic Glucose-Enhanced (DGE) MRI: Translation to Human Scanning and First Results in Glioma Patients," Tomography, vol. 1, no. 2, p. 105, Dec. 2015.
[25] L. Knutsson et al., "Arterial Input Functions and Tissue Response Curves in Dynamic Glucose-Enhanced (DGE) Imaging: Comparison between glucoCEST and Blood Glucose Sampling in Humans," Tomogr. 2018, Vol. 4, Pages 164-171, vol. 4, no. 4, pp. 164-171, Dec. 2018.
[26] F. Schweser, S. D. Robinson, L. de Rochefort, W. B. Li, and Kristian, "An illustrated comparison of processing methods for phase MRI and QSM: removal of background field contributions from sources outside the region of interest," Wiley Online Libr., vol. 30, no. 4, Apr. 2017.
[27] F. Schweser, A. Deistung, and J. R. Reichenbach, "Foundations of MRI phase imaging and processing for Quantitative Susceptibility Mapping (QSM)," Z. Med. Phys., vol. 26, no. 1, pp. 6- 34, Mar. 2016.
[28] E. Mueller and S. Thesen, "METHOD FOR OPERATING A MAGNETIC RESONANCE TOMOGRAPHY APPARATUS WITH SHIM COIL ADJUSTMENT DEPENDENT ON POSITIONAL CHANGES OF THE IMAGED REGION," US 6,509,735 B2, 2003.
[29] C. N. Ladefoged, L. Marner, A. Hindsholm, I. Law, L. Højgaard, and F. L. Andersen, "Deep learning based attenuation correction of PET/MRI in pediatric brain tumor patients: Evaluation in a clinical setting," Front. Neurosci., vol. 13, no. JAN, pp. 1-9, 2019.
[30] F. Cognolato, K. O'Brien, J. Jin, S. Robinson, F. B. Laun, M. Barth, S. Bollmann, "NeXtQSM - A complete deep learning pipeline for data-consistent quantitative susceptibility mapping trained with hybrid data", arXiv:2107.07752v
[31] W. Grissom et al., "Spatial domain method for the design of RF pulses in multicoil parallel excitation", Magn Res. Med, vol 56, 2006
[32] B. Sutton, D. Noll, and J. Fessler. "Fast, iterative image reconstruction for MRI in the presence of field inhomogeneities." IEEE Transactions on Medical Imaging, 22(2):178-188, 2003
[33] M. Lustig, J. M. Pauly,"SPIRiT: Iterative Self-consistent Parallel ImagingReconstruction From Arbitraryk-Space" Magnetic Resonance in Medicine 64:457-471 (2010)
[34] T. Liu, P. Spincemaille P, L. de Rochefort, B. Kressler, Y. Wang Y, "Calculation of susceptibility through multiple orientation sampling (COSMOS): a method for conditioning the inverse problem from measured magnetic field map to susceptibility source image in MRI." Magn. Reson. Med., January 2009;61(1):196-204.
[35] L. Cordero-Grande, E. J. Hughes, J. Hutter, A. N. Price, and J. V Hajnal, "Three-dimensional motion corrected sensitivity encoding reconstruction for multi-shot multi-slice MRI: Application to neonatal brain imaging," Magn. Reson. Med., vol. 79, no. 3, pp. 1365-1376, 2018
[36] K. P. Pruessmann, M. Weiger, M. B. Scheidegger, and P. Boesiger, "SENSE: sensitivity encoding for fast MRI," Magn. Reson. Med., vol. 42, no. 5, pp. 952-962, 1999
[37] M. Lustig et al. "Sparse MRI: The application of compressed sensing for rapid MR imaging" Magnetic Resonance in Medicine, 58:1182-1195, 2007
[38] Staib, L.H.; Duncan, J.S. (1992). "Boundary finding with parametrically deformable models". IEEE Transactions on Pattern Analysis and Machine Intelligence. 14 (11): 1061-1075.

## Claims

1. A computer-implemented method for generating a subject-specific map (20) of a magnetic susceptibility of a region of interest within a subject (62), the method comprising the steps of:
- Receiving an MR image (2) of the region of interest;
- Feeding the MR image (2) into an algorithm (4, 6),
wherein the algorithm (4, 6) comprises at least one trained artificial neural network, wherein the output of the algorithm (4, 6) is an output image having improved contrast between bone and air, wherein the output image of the algorithm (4, 6) is a synthetic CT image (10), ;
- Segmenting (12) the output image into air and at least two types of tissue, namely at least bone and at least one type of soft tissue, to obtain a segmented image;
- Assigning (14) pre-determined values for the magnetic susceptibility to each category of tissue and air in the segmented image to obtain the subject-specific map of the magnetic susceptibility.

2. The method of claim 1, wherein the method comprises the further step of estimating (40) a map of the demagnetization field (42) caused by the subject when placed in a B₀ magnetic field from the map of magnetic susceptibility.

3. A computer program comprising instructions which, when the program is executed by a computer (70), cause the computer to carry out the method of one of the claims 1 to 2.

4. A system for generating a subject-specific map of a magnetic susceptibility of a region of interest within a subject, the system comprising:
a first interface, configured for receiving an MR image (2) of the region of interest;
a computational unit, configured for
- feeding the MR image (2) as input into an algorithm (4, 6), wherein the algorithm (4, 6) comprises at least one trained artificial neural network, wherein the output of the algorithm (4, 6) is an output image (8, 10) having improved contrast between bone and air, wherein the output image of the algorithm (4, 6) is a synthetic CT image (10);
- segmenting (12) the output image (8, 10) into air and at least two types of tissue, namely at least bone and at least one type of soft tissue, to obtain a segmented image;
- assigning (14) pre-determined values (16) for the magnetic susceptibility to each category of tissue and air in the segmented image to obtain the subject-specific map (20) of the magnetic susceptibility; and
a second interface, configured for providing the map (20) of the magnetic susceptibility.

## Patentansprüche

1. Computerimplementiertes Verfahren zum Erzeugen einer patientenspezifischen Karte (20) einer magnetischen Suszeptibilität einer Interessensregion innerhalb eines Patienten (62), wobei das Verfahren die folgenden Schritte umfass:
- Empfangen eines MR-Bildes (2) der Interessensregion;
- Einspeisen des MR-Bildes (2) in einen Algorithmus (4, 6), wobei der Algorithmus (4, 6) mindestens ein trainiertes künstliches neuronales Netzwerk umfasst, wobei die Ausgabe des Algorithmus (4, 6) ein Ausgabebild mit verbessertem Kontrast zwischen Knochen und Luft ist, wobei das Ausgabebild des Algorithmus (4, 6) ein synthetisches CT-Bild (10) ist;
- Segmentieren (12) des Ausgabebildes in Luft und mindestens zwei Gewebearten, nämlich mindestens Knochen und mindestens eine Art von Weichgewebe, um ein segmentiertes Bild zu erhalten;
- Zuordnen (14) vorbestimmter Werte für die magnetische Suszeptibilität für jede Kategorie von Gewebe und Luft in dem segmentierten Bild, um die patientenspezifische Karte der magnetischen Suszeptibilität zu erhalten.

2. Verfahren nach Anspruch 1, wobei das Verfahren den weiteren Schritt des Schätzens (40) einer Karte des Entmagnetisierungsfeldes (42), das durch den Patienten erzeugt wird, wenn er in einem B₀-Magnetfeld platziert wird, aus der Karte der magnetischen Suszeptibilität umfasst.

3. Computerprogramm, umfassend Anweisungen, die bei Ausführung des Programms durch einen Computer (70) den Computer zum Durchführen des Verfahrens nach einem der Ansprüche 1 bis 2 veranlassen.

4. System zum Erzeugen einer patientenspezifischen Karte einer magnetischen Suszeptibilität einer Interessensregion innerhalb eines Patienten, wobei das System Folgendes umfasst:
eine erste Schnittstelle, die zum Empfangen eines MR-Bildes (2) der Interessensregion ausgelegt ist;
eine Recheneinheit, die zu Folgendem ausgelegt ist:
- Einspeisen des MR-Bildes (2) als eine Eingabe in einen Algorithmus (4, 6), wobei der Algorithmus (4, 6) mindestens ein trainiertes künstliches neuronales Netzwerk umfasst, wobei die Ausgabe des Algorithmus (4, 6) ein Ausgabebild (8, 10) mit verbessertem Kontrast zwischen Knochen und Luft ist, wobei das Ausgabebild des Algorithmus (4, 6) ein synthetisches CT-Bild (10) ist;
- Segmentieren (12) des Ausgabebildes (8, 10) in Luft und mindestens zwei Gewebearten, nämlich mindestens Knochen und mindestens eine Art von Weichgewebe, um ein segmentiertes Bild zu erhalten;
- Zuordnen (14) vorbestimmter Werte (16) für die magnetische Suszeptibilität für jede Kategorie von Gewebe und Luft in dem segmentierten Bild, um die patientenspezifische Karte (20) der magnetischen Suszeptibilität zu erhalten; und
eine zweite Schnittstelle, die zum Bereitstellen der Karte (20) der magnetischen Suszeptibilität ausgelegt ist.

## Revendications

1. Un procédé mis en œuvre par ordinateur pour créer une carte (20) spécifique à un sujet d'une susceptibilité magnétique d'une région à laquelle on s'intéresse dans un sujet (62), le procédé comprenant les stades de :
- réception d'une image (2) RM de la région à laquelle on s'intéresse ;
- introduction de l'image (2) RM dans un algorithme (4, 6), dans lequel l'algorithme comprend au moins un réseau neuronal artificiel entraîné, dans lequel la sortie de l'algorithme (4, 6) est une image de sortie ayant un contraste amélioré entre l'os et l'air, dans lequel l'image de sortie de l'algorithme (4, 6) est une image (10) CT synthétique ;
- segmentation de l'image (12) de sortie en air et en au moins deux types de tissu, à savoir au moins os et au moins un type de tissu mou, pour obtenir une image segmentée ;
- affectation (14) de valeurs déterminées à l'avance de la susceptibilité magnétique à chaque catégorie de tissu et d'air dans l'image segmentée pour obtenir la carte spécifique au sujet de la susceptibilité magnétique.

2. Le procédé de la revendication 1, dans lequel le procédé comprend le stade supplémentaire d'estimation (40) d'une carte du champ (42) de démagnétisation provoquée par le sujet, lorsqu'il est placé dans un champ magnétique B₀ à partir de la carte de susceptibilité magnétique.

3. Programme d'ordinateur comprenant des instructions qui, lorsque le programme est exécuté par un ordinateur (70), font que l'ordinateur effectue le procédé suivant l'une des revendications 1 à 2.

4. Un système pour créer une carte spécifique à un sujet de susceptibilité magnétique d'une région à laquelle on s'intéresse dans un sujet, le système comprenant :
une première interface, configurée pour recevoir une image (2) RM de la région à laquelle on s'intéresse ;
une unité informatique, configurée pour
- envoyer l'image (2) RM comme entrée dans un algorithme (4, 6), dans lequel l'algorithme (4, 6) comprend au moins un réseau neuronal artificiel entraîné, dans lequel la sortie de l'algorithme (4, 6) est une image (8, 10) de sortie ayant un contraste amélioré entre l'os et l'air, dans lequel l'image de sortie de l'algorithme (4, 6) est une image (10) CT synthétique ;
- segmenter (12) l'image (8, 10) de sortie en air et en au moins deux types de tissu, à savoir au moins un os et au moins un type de tissu mou, pour obtenir une image segmentée ;
- affecter (14) des valeurs (16) déterminées à l'avance de la susceptibilité magnétique à chaque catégorie de tissu et d'air dans l'image segmentée pour obtenir la carte (20) spécifique au sujet de la susceptibilité magnétique ; et
une deuxième interface configurée pour donner la carte (20) de la susceptibilité magnétique.
